# EUROPEAN PATENT SPECIFICATION

(11) **EP 2 741 754 B1**
(45) Date of publication and mention of the grant of the patent: **27.05.2015**
(21) Application number: 12735548.5
(22) Date of filing: 17.07.2012
(51) Int. Cl.: A61K 31/715, A61K 36/48, A61K 9/00, A61K 9/08, A61P 11/00

(54) **USE OF A POLYSACCHARIDE POLYMER FROM THE SEEDS OF THE TAMARIND TREE IN PROTECTING AGAINST TOBACCO-ASSOCIATED DAMAGES**
VERWENDUNG VON TAMARINDSAMENGUMMI ZUM SCHUTZ VOR TABAK-INDUZIERTEN SCHÄDEN
UTILISATION DE POLYSACCHARIDE DE GRAINE DE TAMARIN POUR PROTÉGER CONTRE UN DOMMAGE LIÉ AU TABAC

(30) Priority: 12.08.2011 EP 11006634
(43) Date of publication of application: 18.06.2014
(73) Proprietor: Zambon S.p.A., 20091 Bresso MI (IT)
(72) Inventor: SARDINA, Marco, I-21040 Gerenzano (VA) (IT); ALETTI, Adonella, I-20900 Monza (IT); MELLONI, Elsa, I-20162 Milano (IT)
(74) Representative: Allaix, Roberto
(86) International application number: PCT/EP2012/063975
(87) International publication number: WO 2013/023856

(56) References cited:
- WO-A1-97/28787
- WO-A1-2012/007113

## Description

### FIELD OF THE INVENTION

The present invention relates to the use of polysaccharide polymer obtained from the seeds of the tamarind tree for protecting the epithelial cells of the respiratory tract by damages induced by tobacco smoke.

Especially, the present invention relates to the use of polysaccharide polymer obtained from the seeds of the tamarind tree for protecting the airway epithelial cells of the respiratory airways from damages induced by smoke of cigarettes, cigars or pipes.

### DESCRIPTION OF THE INVENTION

According to R. Kulkarni et al. (Infection and Immunity, May 2010, p. 2146-2152) tobacco and, in particular, cigarette smoke and exposure to second-hand smoke are important risk factors for a variety of systemic and respiratory tract diseases that include six of the eight leading causes of death in the world (WHO. 2008. WHO report on global tobacco epidemic, 2008: the MPOWER package. World Health Organization, Geneva, Switzerland). Today, tobacco kills approximately 5 million people every year worldwide, and this number is estimated to rise to 8.3 million by 2030 (Mathers, C. D., and D. Loncar. 2006. Projections of global mortality and burden of disease from 2002 to 2030. PLoS Med. 3:e442).

Smokers are at particular risk of upper and lower respiratory tract infections.

The human nasopharyngeal epithelium, a sentinel site of respiratory tract immunity, is constantly exposed to microbial patterns and other environmental stimuli.

In particular, cigarette smoke can be considered one of the most dangerous environmental stresses, bringing about structural changes in the respiratory tract as well as alterations in immune responses that may lead to predisposition to infection or exacerbation of existing pathologies.

The gaseous and soluble phases of cigarette smoke contribute to the pathogenesis of chronic obstructive pulmonary disease (COPD). Chronic oxidative stress of cigarette smoking induces mucus secretion and inhibits cystic fibrosis transmembrane conductance regulator function. The increased mucus viscosity renders the airways susceptible to bacterial infections, a hallmark of chronic bronchitis.

Damage to airway epithelial cells by tobacco smoke, particularly cigarette smoke, is therefore intimately related to the pathogenesis of a variety of acute and chronic respiratory disorders.

Tamarind tree is widespread in India, Africa and in the South East Asia. In Middle Eastern countries, tamarind juice from the tamarind fruit can be a drink prepared by infusing dried tamarind pulp. Tamarind can also be useful for the preservation of food products and as a sauce in recipes.

The tamarind seed finds various applications, once ground to the powder form (known as "tamarind gum" or "tamarind kernel powder"). Commercially available tamarind kernel powder can be employed as thickener and a sizing agent in textile and paper industries; and as thickening, gelling, stabilizing and binding agent in food and pharmaceutical industries.

General properties, chemical composition and chemical structure of tamarind kernel powder and of tamarind seed polysaccharide can be found in: Gupta V, Puri R, Gupta S, Jain S, Rao GK.; Tamarind kernel gum: an upcoming natural polysaccharide. Syst Rev Pharm; 2010; 1:50-4.

It has now surprisingly found that a polysaccharide polymer obtained from the seeds of the tamarind tree provides protection to airway epithelial cells against tobacco smoke-induced damages, by preserving epithelial barrier integrity and hence, preventing structural changes in the respiratory tract that may lead to predisposition to infection or exacerbation of existing acute and chronic respiratory disorders.

It is therefore a first object of the present invention a polysaccharide polymer obtained from the seeds of the tamarind tree (tamarind seed polysaccharide), for use in protecting airway epithelial cells against tobacco smoke-induced damages.

More specifically, the present invention relates to the protection of airway epithelial cells damages caused by cigarette smoke, through the administration of a polysaccharide polymer obtained from the seeds of the tamarind tree (tamarind seed polysaccharide).

The local administration to the epithelial cells of the respiratory airways of a smoker or a second-hand smoker of a preparation containing the tamarind seed polysaccharide according to the invention plays an effective role in protecting airway epithelial cells against tobacco smoke-induced damages, particularly cigarette smoke-induced damages.

Tamarind seed polysaccharide is a nonionic, neutral, branched polysaccharide comprising a cellulose-like backbone substituted by xylose (α 1→6) and galacto (β 1→2)xylose (α 1→6) substituents (Lang P. et al., Macromolecules, 1993, 26, 3992-3998).

Tamarind seed polysaccharide suitable for the preparations according to the present invention may be obtained according to any method known in the art, preferably following the methods described in WO 97/28787.

To achieve the desired effect, a polysaccharide polymer obtained from the seeds of the tamarind tree according to the invention can be locally administered preferably as an aerosolized liquid preparation, by suitable commercially available inhalation devices, to the mucous membranes of the respiratory airways of a cigarette smoker or a second-hand smoker (herein also named as passive-smoker), namely a person who involuntarily takes environmental smoked tobacco of a cigarette smoker.

It is therefore another object of the present invention a preparation in the form of a liquid dosage for topical application, particularly aerosolized topical application, which comprises a polysaccharide polymer obtained from the seeds of the tamarind tree, said preparation being useful in protecting airway epithelial cells against tobacco smoke-induced damages, particularly cigarette smoke-induced damages. The preparation according the invention contains from 0.25 to 0.5 w/v% (hereunder the unit "w/v%" is referred to as "%" for simplicity) of the polysaccharide polymer obtained from the seeds of the tamarind tree. Preferably, the preparation according the invention contains 0.3% of the polysaccharide polymer obtained from the seeds of the tamarind tree.

The preparation according to the invention can further comprise pH adjusting agents well known to a person skilled in the art, including pharmaceutically acceptable acids or bases and buffering agents. For example, the acids may include one or more inorganic mineral acids such as hydrochloric, hydrobromic, sulphuric, phosphoric and nitric acid, or one or more organic acids such as acetic, succinic, tartaric, ascorbic, citric, glutamic, benzoic, methanesulphonic, ethanesulfonic and trifluoroacetic acid. The bases may include inorganic bases and organic bases, such as alkaline carbonate, alkaline bicarbonate, alkaline earth metal carbonate, alkaline hydroxide and alkaline earth metal hydroxide. For example, the inorganic base may be an alkaline hydroxide such as lithium hydroxide, potassium hydroxide, cesium hydroxide or sodium hydroxide; an alkaline carbonate may be calcium carbonate or sodium carbonate; an alkaline bicarbonate may be sodium bicarbonate. For example, buffering agents include a phosphate buffer such as sodium dihydrogen phosphate commonly termed monosodium phosphate (NaH₂PO₄) and disodium hydrogen phosphate commonly termed disodium phosphate (Na₂HPO₄), an acetate buffer (sodium acetate-acetic acid system) a citrate buffer (sodium citrate-citric acid system) and mixtures thereof.

In a particular aspect of the present invention, the buffering agent is preferably selected from monosodium phosphate (NaH₂PO₄), disodium phosphate (Na₂HPO₄) or mixture thereof, and sodium citrate-citric acid system.

The type and concentration of the pH adjusting agents suitable for use in the disclosed preparation would be dependent on the desired pH value which ranges within 6.0 to 8.0, preferably within 6.8 and 7.5.

The isotonicity of the preparation vehicle is preferably adjusted by sodium chloride.

The local administration to the epithelial cells of the respiratory airways of a smoker or a second-hand smoker of the preparation according to the invention can be carried out by an aerosol delivery system. The aerosolized liquid preparation is therefore sufficiently mobile to coat a wide area of the airway epithelial cells of the respiratory tract, thus providing protective effect to epithelial barrier integrity.

Preferably, the aerosol delivery system is a commercially available nebulizer, such as a jet nebulizer, which accepts the liquid preparation and transforms said liquid preparation into fine droplets, so that it can be easily inhaled through a mouthpiece or a face mask.

As an example, when the jet nebulizer is turned on, a jet of air is forced through the preparation according to the invention, turning it into fine droplets which are moved along a tube. The subject inhales the preparation, through a mouthpiece or a face mask. A face mask is preferably used for pediatric subjects.

The amount of the preparation administered to a subject may be dependent on a variety of factors, including the general quality of health, age, gender, weight of the subject in need thereof. For example, from 6 to 18 mg/day of tamarind seed polysaccharide can be administered to a subject in need thereof.

A process for preparing a preparation as described above can be carried out according to methods well known to a person skilled in the art; for example, said process comprises dissolving the tamarind seed polysaccharide in purified water such as water for injections and adding a pH adjusting agent until the desired pH is reached.

As used herein, the singular forms "a," "an" and "the" include plural referents unless the context clearly states otherwise. Thus, for example, reference to "a polysaccharide polymer obtained from the seeds of the tamarind tree" includes mixtures of polysaccharide polymers obtained from the seeds of the tamarind tree. As used herein, the term "airway epithelial cells" means epithelial cells of the respiratory airways.

As used herein, the term "tobacco smoke" includes beedi, cigar, filtered and unfiltered cigarette, pipe, hookah and kretek smoke.

As used herein, the term "alleviation" includes mitigation, decrease, diminution and reduction.

As used herein, the term "inhalation" or "administration by inhalation" refers to the introduction into the respiratory organs, especially into and/or via the airways, preferably into and/or via the nasal cavity and oral cavity.

As used herein, the term "nebulizer" includes jet nebulizers.

As used herein, the term "polysaccharide polymer obtained from the seeds of the tamarind tree", interchangeable with the term "tamarind seed polysaccharide" and abbreviated as "TSP", means any polysaccharide-enriched fraction obtainable from tamarind kernel powder currently available on the market. A partially purified polysaccharide fraction of tamarind gum is sold, for instance, by Dainippon Pharmaceutical Co. LTD of Osaka, Japan, under the trade name Glyloid^{®}. For the purpose of the present invention, however, the concerned polysaccharide is preferably obtained following any method known in the art to give a practically pure tamarind seed polysaccharide from commercial tamarind gum sources.

As used herein, the term "preparation" is intended in a broader sense to include not only all conventional pharmaceutical preparations but also preparations useful as diet supplements, medical devices and food additives.

As used herein, the term "subject" refers to a tobacco smoker, especially a cigarette smoker, or a second-hand smoker, namely a person who involuntarily takes environmental smoked tobacco of a tobacco smoker, especially a cigarette smoker. The following Examples illustrates but do not limit the present invention.

### Example 1

Cigarette Smoke Condensate (CSC) is able to impair integrity and induce injury of airway epithelial cells.

The efficacy of TSP in protecting airway epithelial cells from damages induced by CSC was investigated *in vitro* on Calu-3 human cell line.

The necessary amount of TSP was diluted in culture medium to reach the 0.3% (g/ml) concentration.

Calu-3 cells were exposed for 24 h to TSP 0.3% alone, to a cigarette smoke-induced injury alone (CSC 5%), or pre-incubated with TSP 0.3% for 1 hr before exposure to CSC 5% for 24h.

At the end of treatments, cell viability was determined by the Trypan Blue dye exclusion test, according to standard technique.

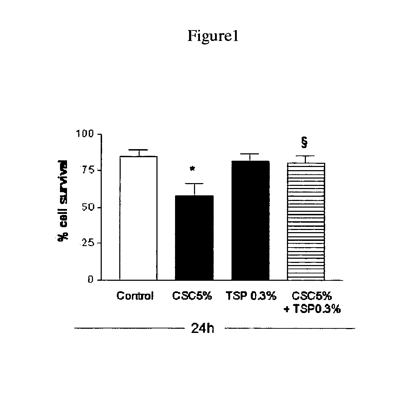

As reported in Fig. 1, the percentage of cell survival was significantly lower in cultures exposed to CSC 5% as compared to control cultures grown in medium alone (59.95% and 83.08%, respectively; p<0.05), whereas, surprisingly, the toxic effect of CSC was significantly prevented by pre-incubation of cells with 0.3% TSP (p<0.05) since the percentage of cell survival on Calu-3 cultured with TSP alone or in association with CSC was similar to that reported for cell control (81.96% and 76.19%, respectively).

**Fig. 1** shows Calu-3 survival cells after 24h of treatment with CSC 5% and/or TSP 0.3% by the Trypan Blue exclusion test. The data, representing the results of five experiments in triplicate, are expressed as the means ± SEM. * = p<0.05 *vs* control cultures and § = p<0.05 *vs* CSC-treated samples.

The above-results demonstrate that TSP may contribute to the protection of the airway epithelium barrier against cigarette smoke-induced injury, by preserving epithelial barrier integrity.

### Example 2

The necessary amount of tamarind seed polysaccharide (TSP) was weighted into a suitable vessel, water for injection was added up to the final volume (100 ml) until complete dissolution of the TSP. The isotonicity of the preparation was adjusted by NaCl and pH adjusting agents were then added to maintain the pH within physiological pH (between 6.8 and 7.5). The so obtained preparation had the following composition:

| | |
|---|---|
| TSP | 0.3% |
| NaCl | 0.9%, |
| Na₂HPO₄ | 0.9%, |
| NaH₂PO₄ | 0.09% |
| Water for injection | to 100 ml |

Following analogous procedure, the below preparation was obtained:

| | |
|---|---|
| TSP | 0.3% |
| NaCl | 0.9%, |
| Citric acid | 0.03%, |
| Sodium citrate | 0.5% |
| Water for injection | to 100 ml |

## Claims

1. A polysaccharide polymer obtained from the seeds of the tamarind tree (tamarind seed polysaccharide), for use in protecting airway epithelial cells against tobacco smoke-induced damages.

2. Polysaccharide polymer for use according to claim 1 wherein tobacco smoke is cigarette smoke.

3. Polysaccharide polymer for use according to claim 2, wherein protection of airway epithelial cells against cigarette smoke-induced damages contributes to prevention of pathogenesis of the cigarette smoke-induced variety of acute and chronic respiratory disorders in cigarette smokers.

4. Polysaccharide polymer for use according to claim 2, wherein protection of airway epithelial cells against cigarette smoke-induced damages contributes to alleviation of the harm of the second-hand smoke to passive-smokers.

5. Polysaccharide polymer for use according to claim 1, wherein the polymer is locally administered to a subject in need thereof as a topical preparation.

6. Polysaccharide polymer for use according to claim 5, wherein the topical preparation is a preparation in the form of a liquid dosage form for topical application.

7. Polysaccharide polymer for use according to claim 6, wherein the liquid dosage form for topical application is an aerosolized liquid dosage form for topical application.

8. Polysaccharide polymer for use according to claim 5, wherein the preparation contains from 0.25 to 0.5% of the polysaccharide polymer obtained from the seeds of the tamarind tree.

9. Polysaccharide polymer for use according to claim 8 wherein the preparation contains 0.3% of the polysaccharide polymer obtained from the seeds of the tamarind tree.

10. Polysaccharide polymer for use according to claim 9, wherein the preparation further comprises a pH adjusting agent.

11. Polysaccharide polymer for use according to claim 10, wherein the pH adjusting agent is a buffering agent selected from monosodium phosphate (NaH₂PO₄), disodium phosphate (Na₂HPO₄), sodium citrate-citric acid system or mixture thereof.

12. A preparation in the form of a liquid dosage for topical application, which comprises a polysaccharide polymer obtained from the seeds of the tamarind tree, for use in protecting airway epithelial cells against tobacco smoke-induced damages.

## Patentansprüche

1. Aus den Samen des Tamarindenbaums gewonnenes Polysaccharidpolymer (Tamarindensamen-Polysaccharid), zur Verwendung zum Schutz von Atemwegsepithelzellen gegen Tabakrauch-induzierte Schäden.

2. Polysaccharidpolymer zur Verwendung nach Anspruch 1, wobei der Tabakrauch Zigarettenrauch ist.

3. Polysaccharidpolymer zur Verwendung nach Anspruch 2, wobei der Schutz der Atemwegsepithelzellen gegen Zigarettenrauch-induzierte Schäden der Vorbeugung der Pathogenese Zigarettenrauch-induzierter akuter und chronischer Atemwegserkrankungen von Rauchern dient.

4. Polysaccharidpolymer zur Verwendung nach Anspruch 2, wobei der Schutz der Atemwegsepithelzellen gegen Zigarettenrauch-induzierte Schäden der Verminderung der Beeinträchtigung von Passivrauchern durch Rauch aus zweiter Hand dient.

5. Polysaccharidpolymer zur Verwendung nach Anspruch 1, wobei das Polymer einer betroffenen Person lokal als topische Zubereitung verabreicht wird.

6. Polysaccharidpolymer zur Verwendung nach Anspruch 5, wobei die topische Zubereitung eine Zubereitung in Form einer flüssigen Darreichungsform zur topischen Anwendung ist.

7. Polysaccharidpolymer zur Verwendung nach Anspruch 6, wobei die flüssige Darreichungsform für die topische Anwendung eine aerosolierte, flüssige Darreichungsform für die topische Anwendung ist.

8. Polysaccharidpolymer zur Verwendung nach Anspruch 5, wobei die Zubereitung 0,25 bis 0,5 % des aus den Samen des Tamarindenbaums gewonnenen Polysaccharidpolymers enthält.

9. Polysaccharidpolymer zur Verwendung nach Anspruch 8, wobei die Zubereitung 0,3 % des aus den Samen des Tamarindenbaums gewonnenen Polysaccharidpolymers enthält.

10. Polysaccharidpolymer zur Verwendung nach Anspruch 9, wobei die Zubereitung außerdem eine Substanz zur Einstellung des pH-Wertes umfasst.

11. Polysaccharidpolymer zur Verwendung nach Anspruch 10, wobei die Substanz zur Einstellung des pH-Werts ein unter Mononatriumphosphat (NaH₂PO₄), Dinatriumphosphat (Na₂HPO₄), einem Natriumcitrat-Zitronensäure-System oder Gemischen davon ausgewählter Puffer ist.

12. Zubereitung in flüssiger Darreichungsform zur topischen Anwendung, die ein aus den Samen des Tamarindenbaums gewonnenes Polysaccharidpolymer umfasst, zur Verwendung zum Schutz der Atemwegsepithelzellen gegen Tabakrauch-induzierte Schäden.

## Revendications

1. Polymère de polysaccharide obtenu à partir de graines de tamarinier (polysaccharide de graines de tamarin), pour utilisation dans la protection des cellules épithéliales aériennes contre les dommages induits par la fumée de tabac.

2. Polymère de polysaccharide pour utilisation selon la revendication 1, dans lequel la fumée de tabac est de la fumée de cigarette.

3. Polymère de polysaccharide pour utilisation selon la revendication 2, dans lequel la protection des cellules épithéliales aériennes contre les dommages induits par la fumée de cigarette contribue à prévenir la pathogenèse des divers troubles respiratoires aigus et chroniques induits par la fumée de cigarette chez les fumeurs de cigarettes.

4. Polymère de polysaccharide pour utilisation selon la revendication 2, dans lequel la protection des cellules épithéliales aériennes contre les dommages induits par la fumée de cigarette contribue à soulager les nuisances de la fumée de seconde main pour les fumeurs passifs.

5. Polymère de polysaccharide pour utilisation selon la revendication 1, lequel polymère est administré localement à un sujet en ayant besoin sous la forme d'une préparation à usage topique.

6. Polymère de polysaccharide pour utilisation selon la revendication 5, dans lequel la préparation à usage topique est une préparation sous la forme d'une forme posologique liquide pour administration par voie topique.

7. Polymère de polysaccharide pour utilisation selon la revendication 6, dans lequel la forme posologique liquide pour application par voie topique est une forme posologique liquide en aérosol pour application par voie topique.

8. Polymère de polysaccharide pour utilisation selon la revendication 5, dans lequel la préparation contient de 0,25 à 0,5 % du polymère de polysaccharide obtenu à partir des graines de tamarinier.

9. Polymère de polysaccharide pour utilisation selon la revendication 8, dans lequel la préparation contient 0,3 % du polymère de polysaccharide obtenu à partir des graines de tamarinier.

10. Polymère de polysaccharide pour utilisation selon la revendication 9, dans lequel la préparation comprend en outre un agent d'ajustement du pH.

11. Polymère de polysaccharide pour utilisation selon la revendication 10, dans lequel l'agent d'ajustement du pH est un agent tampon choisi parmi le phosphate monosodique (NaH₂PO₄) le phosphate disodique (Na₂HPO₄), un système de citrate de sodium-acide citrique, ou un mélange de ceux-ci.

12. Préparation sous la forme d'un dosage liquide pour application par voie topique, qui comprend un polymère de polysaccharide obtenu à partir de graines de tamarinier, pour utilisation dans la protection des cellules épithéliales aériennes contre les dommages induits par la fumée de tabac.
